# EUROPÄISCHE PATENTANMELDUNG

(11) **EP 2 241 252 A1**
(43) Veröffentlichungstag der Anmeldung: **20.10.2010**
(21) Anmeldenummer: 09155318.0
(22) Anmeldetag: 17.03.2009
(51) Int. Cl.: A61B 5/15

(54) **Testvorrichtung insbesondere für Blutzuckertests**

(71) Anmelder: F. Hoffmann-La Roche AG, 4070 Basel (CH); Roche Diagnostics GmbH, 68305 Mannheim (DE)
(72) Erfinder: Hoenes, Dr. Joachim, D-64673 Zwingenberg (DE)
(74) Vertreter: Pfiz, Thomas

(57) **Zusammenfassung**

Die Erfindung betrifft eine Testvorrichtung insbesondere für Blutzuckertests mit einem Stechelement (14), das mit einem Stechteil (16) zum Erzeugen eines Hauteinstichs (34) versehen ist, und einem das Stechelement (14) zumindest im Bereich des Stechteils (16) abschirmenden Schutzelement (20). Erfindungsgemäß wird vorgeschlagen, dass das Stechelement (14) eine Sammelstruktur (18) zum Aufnehmen von Körperflüssigkeit aus dem Hauteinstich (34) aufweist, und dass das Schutzelement (20) eine Schutzfolie (26) zum Abdecken der Sammelstruktur (18) und einen Haltekörper (28) zur Halterung der Schutzfolie (26) an dem Stechelement (14) umfasst.

## Beschreibung

Die Erfindung betrifft eine Testvorrichtung insbesondere für Blutzuckertests mit einem Stechelement, das mit einem Stechteil zum Erzeugen eines Hauteinstichs versehen ist, und einem das Stechelement zumindest im Bereich des Stechteils abschirmenden Schutzelement. Die Erfindung betrifft weiter ein Herstellungsverfahren für eine solche Testvorrichtung.

Nadeln bzw. Lanzetten zur Gewinnung von Körperflüssigkeiten für Blutzuckertests müssen bis zum Einstechen in die Haut steril sein. Dazu werden die Nadelspitzen üblicherweise keimdicht verpackt und z.B. durch Bestrahlung sterilisiert. Die Verpackung hält die Spitzen dann steril, bis sie entfernt wird. Bei manuellem Gebrauch ist ein Abdrehen oder Abziehen eines die Spitze umhüllenden Plastikteils üblich. Im Zusammenhang mit automatisierten Stechhilfen ist es aus der WO 01/66010 bekannt, ein die Spitze einbettendes Elastomerteil direkt beim Stechvorgang zu durchstechen.

Ausgehend hiervon liegt der Erfindung die Aufgabe zugrunde, die im Stand der Technik bekannten Erzeugnisse und Fertigungsverfahren weiter zu verbessern und so zu gestalten, dass eine hygienische und einfache Handhabung bei der Herstellung, Lagerung und im Einsatz solcher disposibler Testvorrichtungen gewährleistet ist.

Zur Lösung dieser Aufgabe wird die in den unabhängigen Patentansprüchen angegebene Merkmalskombination vorgeschlagen. Vorteilhafte Ausgestaltungen und Weiterbildungen der Erfindung ergeben sich aus den abhängigen Ansprüchen.

Die Erfindung geht von dem Gedanken aus, eine Verbundstruktur als Schutzelement für ein System mit integrierter Probenaufnahmestruktur einzusetzen, so dass die spezifischen Vorteile der Verbundpartner sich ergänzen. Dementsprechend wird erfindungsgemäß vorgeschlagen, dass das Stechelement eine Sammelstruktur zum Aufnehmen von Körperflüssigkeit aus dem Hauteinstich aufweist, und dass das Schutzelement eine Schutzfolie zum Abdecken der Sammelstruktur und einen an dem Stechelement vorzugsweise mittels eines Formwerkzeugs angeformten, insbesondere angespritzten Haltekörper zur Halterung der Schutzfolie umfasst. Die Sammelstruktur ermöglicht eine Probengewinnung bereits im Zuge des für den Probenzugang erforderlichen Stichs, so dass für den Benutzer das Handling vereinfacht wird. Durch die Schutzfolie wird verhindert, dass angeformtes Material des Schutzelements ungewollt in die Aufnahmestruktur eindringt, aus der es beim Stechvorgang kaum vollständig entfernt werden könnte. Der Haltekörper sorgt für die notwendige Struktursteifigkeit und ermöglicht die Folienhalterung auch im Produktionsprozess sowie einen dichten Anschluss an das Stechelement. Hierbei ist zu bedenken, dass eine alleinige Folienverpackung das komplette Stechelement umschließen müsste, um die notwendige Sterilität zu gewährleisten. Es würde aber einen beträchtlichen Aufwand erfordern, eine solche Verpackung vollständig und in jedem Einzelfall zuverlässig von der Sammelstruktur zu entfernen, so dass die Probenaufnahme und ggf. ein Probentransfer ungehindert erfolgen können.

Vorteilhafterweise ist die Sammelstruktur durch eine mit Körperflüssigkeit befüllbare Ausnehmung des Stechteils insbesondere in Form einer einseitig offenen Rille oder eines beidseitig offenen Durchbruchs gebildet, um die Herstellung zu vereinfachen und auch kleinste Probenmengen aufnehmen zu können, wobei im unbenutzten Zustand des Stechelements die Ausnehmung durch die Schutzfolie überdeckt ist und somit auch die Oberflächeneigenschaften im Lagerzustand kaum beeinträchtigt werden.

Für die Gewinnung wässriger Proben ist es von Vorteil, wenn das Stechelement eine hydrophile Beschichtung aufweist, die durch die Schutzfolie zumindest bereichsweise abgedeckt wird und damit lagerstabil bleibt.

Um die Schutzfunktion beim Einsatz aufzuheben, können die Schutzfolie und der Haltekörper an dem Stechelement gemeinsam beweglich angeordnet oder davon entfernbar sein, so dass im Gebrauchszustand des Stechelements das Stechteil und die Sammelstruktur freigelegt sind.

Hierbei ist es besonders günstig, wenn das Stechteil unter Durchdringung des Haltekörpers und/oder der Schutzfolie in die Haut einstechbar ist.

Eine weitere Verbesserung in diesem Zusammenhang sieht vor, dass das Schutzelement beim Erzeugen des Hauteinstichs einen Anschlag gegenüber der Haut oder einem Widerlager bildet.

Zur einfachen Anpassung an die spezifische Sammelstruktur kann die Schutzfolie eine zweilagige Folienhülle oder eine einlagige Foliendecke bilden.

Aufgrund der Fixierung an der Haltestruktur ist es hinreichend, wenn die Schutzfolie lose auf dem Stechteil aufliegt oder mit diesem lösbar haftend verbunden ist, so dass auch eine ungewollte Verschiebung im Produktionsprozess vermieden wird.

Eine weitere vorteilhafte Ausgestaltung sieht vor, dass der massive Haltekörper mit der Schutzfolie einen festen Verbund bildet, wobei der Haltekörper die Schutzfolie vollständig oder an einem Endabschnitt umhüllt. Eine solche feste Verbindung kann in einem Spritzgussprozess aufgrund der Erhitzung des Materials einfach geschaffen werden.

Weiterhin ist es günstig, wenn der Haltekörper als Spritzgussteil vorzugsweise aus einem Elastomerwerkstoff ausgebildet und unmittelbar an dem Stechelement angespritzt ist. In besonders bevorzugter Ausführung ummantelt der Haltekörper einen Schaft des Stechelements.

Um eine zuverlässige Fixierung und sterile Abschirmung zu erreichen, ist es vorteilhaft, wenn der Haltekörper vorzugsweise im Bereich einer über die Schutzfolie hinausgreifenden Randpartie keim- bzw. sterildicht mit dem Stechelement verbunden ist, so dass Keime bis unmittelbar vor Benutzung nicht an das Stechteil vordringen können. Das Schutzelement erhält somit die Sterilität des Stechteils im unbenutzten Zustand aufrecht und/oder ermöglicht das hygienische Entsorgen zumindest des Stechteils im benutzten Zustand. Eine solche keimdichte Verbindung wird durch eine direkt haftende Werkstoffverbindung geschaffen, so dass im Dichtbereich keine Spalten, Fugen, Löcher oder sonstige Undichtigkeiten im Bereich von mehr als 10 Mikrometer Durchlassweite auftreten.

Eine weitere Erhöhung der Systemintegration mit vereinfachter Handhabungsmöglichkeit ergibt sich dadurch, dass die mit Körperflüssigkeit beaufschlagte Sammelstruktur mit einem auf einen Analyten in der Körperflüssigkeit ansprechenden analytischen Testelement durch eine Transferbewegung in Kontakt bringbar ist, so dass Körperflüssigkeit aus der Sammelstruktur direkt auf das Testelement übergeht, ohne dass eine größere Fließstrecke erforderlich ist. In diesem Zusammenhang ist es auch von besonderem Vorteil, wenn der Haltekörper ein Führungsglied zur Bewegungssteuerung des Stechelements bildet.

In verfahrensmäßiger Hinsicht wird die eingangs genannte Aufgabe dadurch gelöst, dass ein Stechelement zumindest im Bereich einer Sammelstruktur mit einer Schutzfolie abgedeckt wird, und dass ein Haltekörper für die Schutzfolie unmittelbar an dem Stechelement angeformt wird. Vorzugsweise wird das Stechelement an einem Schaft ummantelt. Eine solche Struktur lässt sich dann beispielsweise durch Bestrahlung dauerhaft sterilisieren. Für eine Massenfertigung ist es vorteilhaft, wenn eine Vielzahl von Stechelementen in bandförmiger oder ringförmiger Anordnung zumindest im Bereich einer jeweiligen Sammelstruktur mit einer Schutzfolie abgedeckt werden, und anschließend ein Haltekörper für die Schutzfolie an den Stechelementen angeformt wird.

Im Folgenden wird die Erfindung anhand der in der Zeichnung schematisch dargestellten Ausführungsbeispiele näher erläutert. Es zeigen
- Fig. 1: eine Testvorrichtung für Blutzuckertests in Form eines Microsamplers mit einem Schutzelement im Längsschnitt;
- Fig. 2: und 3 jeweils eine weitere Ausführungsform eines Microsamplers mit Schutzelement in verschiedenen Einsatzzuständen;
- Fig. 4: eine Ausführungsform eines in Kombination mit einem Testelement magazinierten Microsamplers in verschiedenen Stadien der Probengewinnung;
- Fig. 5: aufeinanderfolgende Prozessschritte bei der Herstellung von Microsamplern mit Schutzelement.

Die in der Zeichnung dargestellten Testvorrichtungen 10 lassen sich als Microsampler (Mikroprobensammler) gegebenenfalls in Kombination mit einem Testelement 12 in Form eines Einwegteils zum Sammeln und Analysieren einer Körperflüssigkeit wie Blut bzw. Gewebeflüssigkeit in einem Handgerät einsetzen. Sie umfassen zu diesem Zweck ein Stechelement 14, das mit einem distalen Stechorgan bzw. Stechteil 16 zum Einstechen in die Haut und einer Sammelstruktur 18 zum Aufnehmen von Körperflüssigkeit versehen ist, und ein Schutzelement 20 zur Abschirmung des Stechelements 14 zumindest im Bereich des Stechorgans und der Sammelstruktur.

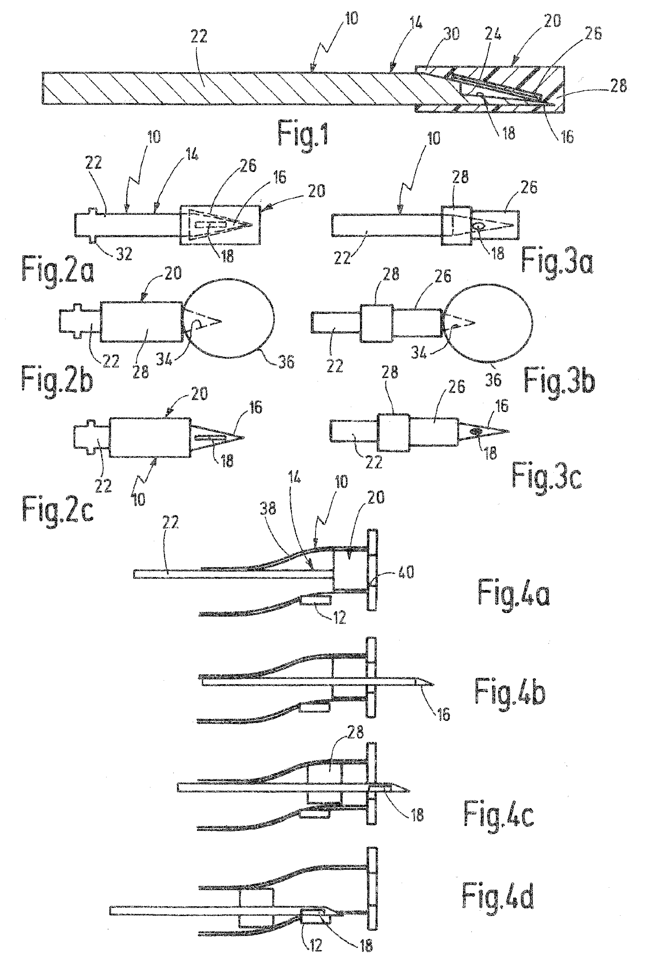

Das in Fig. 1 gezeigte lanzettenartige Stechelement 14 weist einen Schaft 22 auf, an dessen distalem Ende eine Nadelspitze als Stechorgan 16 ausgebildet ist. Die Sammelstruktur 18 ist durch eine axial verlaufende, einseitig offene Kapillarrille im Bereich der Nadelspitze gebildet. Um die Flüssigkeitsaufnahme zu verbessern, ist das metallische Stechelement 14 zumindest im distalen Bereich mit einer hydrophilen Beschichtung 24 versehen.

Im unbenutzten Bereitstellungszustand der Vorrichtung befindet sich das Schutzelement 20 in Wirkstellung über dem distalen Endabschnitt des Stechelements 14. Das Schutzelement 20 besteht aus einer Schutzfolie 26 zur Abdeckung mindestens der Sammelstruktur 18 und aus einem Haltekörper 28 zur Halterung der Schutzfolie 26 an dem Stechelement 14. Die Schutzfolie 26 liegt in dem gezeigten Beispiel nach Fig. 1 als lose oder ggf. leicht haftende einlagige Foliendecke einseitig über der Sammelstruktur 18 auf. Der als Spritzgussteil aus einem Elastomermaterial massiv ausgebildete Haltekörper 28 umhüllt die Schutzfolie 26 vollständig und ist an einem über die Schutzfolie 26 proximal hinausgreifenden Endabschnitt 30 gegen das Eindringen von Keimen dicht mit dem Schaft 22 verbunden. Auf diese Weise lässt sich das Stechelement 14 im Bereich seines Stechteils 16 steril und mechanisch abgeschirmt bereithalten, während die Schutzfolie 26 zumindest das Aufnahmevolumen der Sammelstruktur 18 stoffdicht begrenzt und somit auch die Hydrophilie der Beschichtung 24 über eine längere Lagerzeit erhält.

Das Schutzelement 20 ist an dem Stechelement 14 in axialer Richtung des Schafts 22 verschiebbar angeordnet, so dass in einem Gebrauchszustand das Stechteil 16 und die Sammelstruktur 18 freigelegt werden, wie es nachstehend weiter erläutert wird. Denkbar ist es auch, dass das Schutzelement 20 nach dem Stechvorgang wieder in seine Ausgangsstellung bringbar ist, um eine hygienische und sichere Entsorgung des Stechelements 14 zu ermöglichen.

Die in Fig. 2 gezeigte Ausführungsform unterscheidet sich im Wesentlichen nur dadurch, dass die Schutzfolie 26 als zweilagige Folienhülle bzw. Tasche die Nadelspitze 16 allseitig umhüllt, und dass die Sammelstruktur 18 durch einen quer zur Längsachse des Schafts 22 durchgehenden Schlitz gebildet ist, der somit in dem in Fig. 2a gezeigten Bereitstellungszustand an beiden Seitenöffnungen durch die Schutzfolie 26 abgedeckt ist. Ein an dem Schaft 22 radial abstehendes Koppelteil 32 ermöglich eine Antriebskopplung mit einem nicht gezeigten Stechantrieb für eine hin und her gehende Stechbewegung.

Wie in Fig. 2b nur symbolisch veranschaulicht, kann durch einen Nadelvorschub ein Hauteinstich 34 in einem Körperteil 36, beispielsweise einer Fingerbeere erzeugt werden, wobei das Stechteil 16 durch das Schutzelement 20 hindurchstechbar ist. Hierbei bildet der Haltekörper 28 mit seiner Front einen Anschlag auf der Hautoberfläche. Denkbar ist es auch, dass der Haltekörper 28 an einem geräteseitigen Widerlager zurückgehalten wird. In dem Hauteinstich 34 bzw. im Wundkanal tritt Körperflüssigkeit aus, mit der sich die Sammelstruktur 18 während des Stechvorgangs ggf. unter Kapillarwirkung füllt. Dabei kann das Sammelvolumen auf mikroskopische Mengen, beispielsweise wenige Nanoliter begrenzt werden, um den Vorgang möglichst schmerzarm zu gestalten.

Fig. 2c zeigt den Microsampler 10 im benutzten Zustand mit beim Stechvorgang freigelegter Nadelspitze 16 und zurückgeschobenem Schutzelement 20. In diesem Zustand ist eine direkte Übertragung der Flüssigkeitsprobe aus der Sammelstruktur 18 auf ein Testelement 12 möglich, ohne dass der Benutzer noch weitere Manipulationen vornehmen müsste.

Die in Fig. 3 in entsprechenden Stellungen beim Stechvorgang gezeigte Ausführungsform unterscheidet sich von dem Beispiel nach Fig. 2 im Wesentlichen nur dadurch, dass der Haltekörper 28 die Schutzfolie 26 nur teilweise umhüllt und die Sammelstruktur 18 auf ein kleines Loch beschränkt ist. Hierbei ist der Haltekörper 28 durch eine ringförmige Umspritzung am proximalen Endabschnitt der Schutzfolie 26 angeformt. Die Schutzfolie 26 bildet eine zweilagige Tasche, wobei eine steife Folienlage für Stabilität sorgt und auf der anderen Seite eine dünne Folienlage eine leichte Durchdringung ohne Beschädigung der Spitze 16 ermöglicht. Um eine ungewollte Verletzung mit der Frontseite auszuschließen, wird eine gerade Vorderkante gewählt.

Fig. 4 zeigt ein Ausführungsbeispiel einer Testvorrichtung 10 mit einer Führungskammer 38 als Teil eines Magazins beispielsweise in Form einer Trommel (nicht gezeigt). Das Stechelement ist an seinem proximalen Schaft 22 mit einem Antrieb koppelbar, während die Spitze in dem Schutzelement 20 gelagert ist. Ein Testelement 12 in Form einer trockenchemischen Reagenzschicht ist in Zuordnung zu dem Stechelement 14 an der Führungskammer 38 angeordnet. Die Reagenzschicht reagiert auf Glukose in einer aufgebrachten Blutprobe durch eine Farbänderung, die sich photometrisch nachweisen lässt.

Im Lagerzustand (Fig. 4a) befindet sich das Schutzelement 20 in einer Anschlagstellung vor einer Durchstechöffnung 40 der Führungskammer 38. Beim Stechvorgang (Fig. 4b) wird das Stechelement 14 durch das Schutzelement 20 und die Öffnung 40 hindurch in die Haut eingestochen. Mit gefüllter Sammelstruktur 18 erfolgt anschließend die Rückziehbewegung (Fig. 4c). Der Haltekörper 28 bildet hierbei ein Führungsglied in der Führungskammer 38 für die Steuerung einer Transferbewegung der Sammelstruktur 18 hin zu dem Testelement 12. Die Kontur der Führungskammer ist dabei so gewählt, dass im zurückgezogenen Zustand die Sammelstruktur 18 an das Testelement 12 angedrückt wird, so dass ein Flüssigkeitsübertrag erfolgt (Fig. 4d).

Für die Herstellung der Microsampler 10 als Einwegteile ist eine kostengünstige Massenfertigung zweckmäßig.

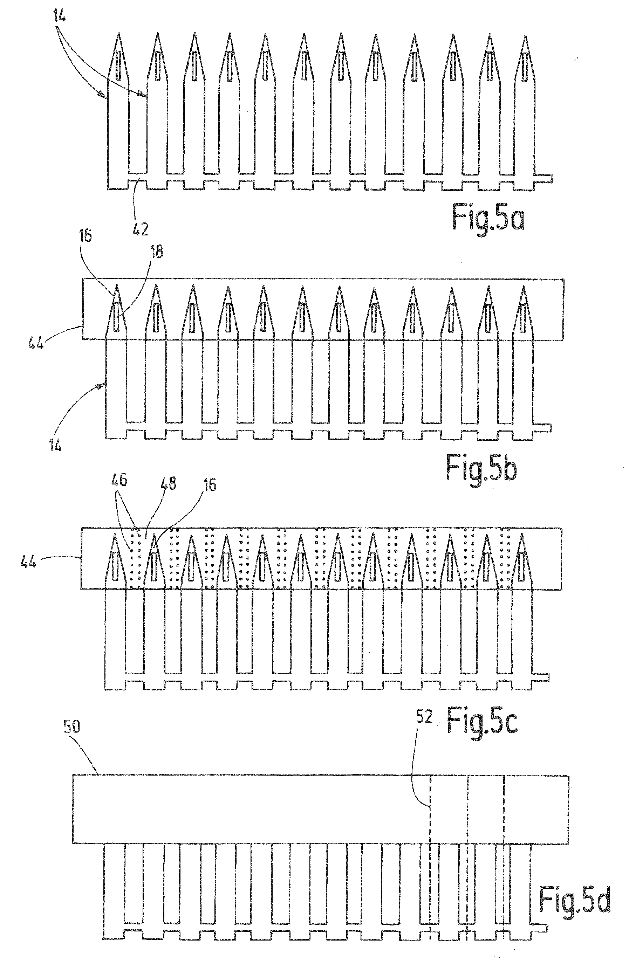

Fig. 5 veranschaulicht verschiedene Fertigungsstadien einer Produktion von Rolle zu Rolle. Gemäß Fig. 5a können die Stechelemente 14 als Flachformteile aus einem Metallband durch Ätzen geformt sein, wobei die einzelnen Elemente durch Brücken 42 miteinander verbunden bleiben. In einem nächsten Schritt wird entsprechend Fig. 5b jeweils ein dünnes Folienband 44 beidseitig über die Nadelspitzen 16 gelegt, so dass auch die Sammelstrukturen 18 überdeckt werden. Sodann werden die Folienbänder 44 gemäß Fig. 5c in den Zwischenräumen zwischen den Nadelspitzen 16 durch Doppelnähte 46 miteinander verbunden, beispielsweise durch Laserschweißen oder Heißsiegeln. Danach wird zwischen den Einzelnähten geschnitten, so dass jede Spitze 16 in einer eigenen Folientasche 48 steckt. Anschließend wird dieser Bereich am Band mit einem Elastomermaterial 50 umspritzt, wie es in Fig. 5d gezeigt ist. Die einzelnen Stechelemente 14 können danach durch Zerteilen der Bandstruktur längs der Schnittlinien 52 erzeugt und ggf. durch Bestrahlung sterilisiert werden.

## Patentansprüche

1. Testvorrichtung insbesondere für Blutzuckertests mit einem Stechelement (14), das mit einem Stechteil (16) zum Erzeugen eines Hauteinstichs (34) versehen ist, und einem das Stechelement (14) zumindest im Bereich des Stechteils (16) abschirmenden Schutzelement (20), **dadurch gekennzeichnet, dass** das Stechelement (14) eine Sammelstruktur (18) zum Aufnehmen von Körperflüssigkeit aus dem Hauteinstich (34) aufweist, und dass das Schutzelement (20) eine Schutzfolie (26) zum Abdecken der Sammelstruktur (18) und einen an dem Stechelement (14) angeformten Haltekörper (28) zur Halterung der Schutzfolie (26) umfasst.

2. Testvorrichtung nach Anspruch 1, **dadurch gekennzeichnet, dass** die Sammelstruktur (18) durch eine mit Körperflüssigkeit befüllbare Ausnehmung des Stechteils (16) insbesondere in Form einer einseitig offenen Rille oder eines beidseitig offenen Durchbruchs gebildet ist, und dass im unbenutzten Zustand des Stechelements (14) die Ausnehmung durch die Schutzfolie (26) überdeckt ist.

3. Testvorrichtung nach Anspruch 1 oder 2, **dadurch gekennzeichnet, dass** das Stechelement (14) eine durch die Schutzfolie (26) zumindest bereichsweise abgedeckte hydrophile Beschichtung (24) aufweist.

4. Testvorrichtung nach einem der Ansprüche 1 bis 3,
**dadurch gekennzeichnet, dass** die Schutzfolie (26) und der Haltekörper (28) an dem Stechelement (14) gemeinsam beweglich angeordnet oder davon entfernbar sind, so dass im Gebrauchszustand des Stechelements (14) das Stechteil (16) und die Sammelstruktur (18) freigelegt sind.

5. Testvorrichtung nach einem der Ansprüche 1 bis 4, **dadurch gekennzeichnet, dass** das Stechteil (16) unter Durchdringung des Haltekörpers (28) und/oder der Schutzfolie (26) in die Haut einstechbar ist.

6. Testvorrichtung nach einem der Ansprüche 1 bis 5, **dadurch gekennzeichnet, dass** das Schutzelement (20) beim Erzeugen des Hauteinstichs (34) einen Anschlag gegenüber der Haut oder einem Widerlager bildet.

7. Testvorrichtung nach einem der Ansprüche 1 bis 6, **dadurch gekennzeichnet, dass** die Schutzfolie (26) eine zweilagige Folienhülle oder eine einlagige Foliendecke bildet.

8. Testvorrichtung nach einem der Ansprüche 1 bis 7,
**dadurch gekennzeichnet, dass** die Schutzfolie (26) lose auf dem Stechteil (16) aufliegt oder mit diesem lösbar haftend verbunden ist.

9. Testvorrichtung nach einem der Ansprüche 1 bis 8, **dadurch gekennzeichnet, dass** der massive Haltekörper (28) mit der Schutzfolie (26) einen festen Verbund bildet, wobei der Haltekörper (28) die Schutzfolie (26) vollständig oder an einem Endabschnitt umhüllt.

10. Testvorrichtung nach einem der Ansprüche 1 bis 9, **dadurch gekennzeichnet, dass** der Haltekörper (28) als Spritzgussteil vorzugsweise aus einem Elastomerwerkstoff ausgebildet und direkt an dem Stechelement (14) angespritzt ist.

11. Testvorrichtung nach einem der Ansprüche 1 bis 10, **dadurch gekennzeichnet, dass** der Haltekörper (28) vorzugsweise im Bereich einer über die Schutzfolie (26) hinausgreifenden Randpartie (30) keimdicht mit dem Stechelement (14) verbunden ist.

12. Testvorrichtung nach einem der Ansprüche 1 bis 11, **dadurch gekennzeichnet, dass** das Schutzelement (20) zum Erhalten der Sterilität zumindest des Stechteils (16) im unbenutzten Zustand und/oder zum hygienischen Entsorgen zumindest des Stechteils (16) im benutzten Zustand ausgebildet ist.

13. Testvorrichtung nach einem der Ansprüche 1 bis 12, **dadurch gekennzeichnet, dass** die mit Körperflüssigkeit beaufschlagte Sammelstruktur (18) mit einem auf einen Analyten in der Körperflüssigkeit ansprechenden analytischen Testelement (12) durch eine Transferbewegung in Kontakt bringbar ist, so dass Körperflüssigkeit aus der Sammelstruktur (18) auf das Testelement (12) übergeht.

14. Testvorrichtung nach einem der Ansprüche 1 bis 13, **dadurch gekennzeichnet, dass** der Haltekörper (28) ein Führungsglied zur Bewegungssteuerung des Stechelements (14) bildet.

15. Verfahren zur Herstellung einer Testvorrichtung (10) nach einem der vorhergehenden Ansprüche bei welchem ein Stechelement (14) zumindest im Bereich einer Sammelstruktur (18) mit einer Schutzfolie (26) abgedeckt wird, und dass ein Haltekörper (28) für die Schutzfolie (26) unmittelbar an dem Stechelement (14) angeformt wird.
